# EUROPEAN PATENT SPECIFICATION

(11) **EP 1 462 079 B1**
(45) Date of publication and mention of the grant of the patent: **23.05.2007**
(21) Application number: 04007121.9
(22) Date of filing: 24.03.2004
(51) Int. Cl.: A61K 6/00

(54) **Dental composition**
Dentalmasse
Composition dentaire

(30) Priority: 28.03.2003 JP 2003090465; 02.06.2003 JP 2003156244
(43) Date of publication of application: 29.09.2004
(73) Proprietor: GC Corporation, Itabashi-ku, Tokyo (JP)
(72) Inventor: Shiniki, Kojima, Tokyo (JP); Akishi, Arita, Tokyo (JP); Go, Mashio, Tokyo (JP); Daisuke, Ota, Tokyo (JP); Daisuke, Usuki, Tokyo (JP)
(74) Representative: Müller-Boré & Partner Patentanwälte

(56) References cited:
- EP-A- 1 287 805
- US-A- 4 259 117

## Description

The present invention relates to a dental composition, which is used as a dental primer, a dental adhesive and a pretreatment agent for adhering a tooth with a filling material, such as a dental composite resin, a dental resin-reinforced cement or the like. More particularly, the present invention relates to the dental composition capable of stably coexisting a polymerizable compound having an acidic group with a reducing agent in a dental composition containing water.

In the field of a dentistry, an adhesive dental composition having strongly adhesive property and easy operation is required in order to adhere the tooth with a dental restorative material, in accordance with the spread of the dental restorative material, such as the dental composite resin or the like. The conventionally used dental composition and its using method are as follows in general, that is, the method comprising, etching the tooth with acids such as phosphoric acid, citric acid or the like, carrying out a primer treatment with a primer containing a polymerizable compound having an adhesive group (an acidic group) for increasing the adhesion, and carrying out a bonding agent treatment with a bonding agent containing a polymerizable monomer not having an acidic group and a polymerization accelerator such as a reducing agent or the like. Moreover, in recent years, the adhesion can be carried out by only the primer treatment and the bonding agent treatment, without the etching treatment. (for example, refer to Japanese Patent Laid Open No. 240712-1991). However, in these methods, at least 2 work steps, i.e. (the etching), the primer treatment and the bonding agent treatment, are necessary as the adhesion operation step as mentioned above.

So, the dental composition being one work step one component type has been required. In order to obtain the effective adhesive strength as the dental composition being one work step one component type, water, the polymerizable compound having the acidic group, and a polymerization catalyst such as a reducing agent and a sensitizer or the like, are at least necessary. For example, as for the polymerization catalyst, it has been carried out in general that a tertiary amine having high biological safety as the reducing agent is combined with camphorquinone being a photopolymerization initiator having an absorption wavelength in a visible light area, as the sensitizer. At this time, although a method using acylphosphine oxides as a photopolymerization catalyst not needing the reducing agent has been considered, since the acylphosphine oxides have a light absorption wavelength in a near ultraviolet area at present, there is a problem that curing is insufficient or not occurs at all by a light irradiator having a light-irradiating wavelength only in a visible light area for considering the biological safety.

However, when the polymerizable compound having the acidic group is coexisted with the reducing agent such as the tertiary amine under the existence of water, there is a problem that a preservation stability is remarkably poor since the composition is cured with time by a chemical reaction, so that it is necessary to package and preserve by dividing the composition into two components or more in fact for the preservation stability. Therefore, since it is necessary to mix each component at the time of using, there is a problem that two or more components must be mixed at the time of using even when the adhesion operation itself is an one work step. That is, the dental composition being the one work step one component type using the reducing agent such as the tertiary amine or the like with water and being not necessary the mixing operation during the period of using, is not obtained yet, thus a new reliable dental composition having high preservation stability with an easy operation has been required. Further, in the pretreatment agent for the dental cement, which contains the reducing agent and the polymerizable compound having the acidic group, the high preservation stability has been similarly required.

The present invention has the object to provide a dental composition, which is not cured with time at the time of preserving even when it is one component in which the polymerizable compound having the acidic group is coexisted with the reducing agent such as the tertiary amine or the like under the existence of water.

The earnest work has been carried out in order to solve the above-mentioned problems and as the result, the method to prevent curing of the composition was found by adding an aluminum oxide powder into a system in which the polymerizable compound having the acidic group is coexisted with the reducing agent such as the tertiary amine or the like under the existence of water, thus this invention was completed.

The present invention relates to a dental composition comprising:
5 to 35 weight parts of (a) a polymerizable compound having an acidic group,
0.1 to 5 weight parts of (b) a reducing agent,
10 to 55 weight parts of (c) water,
0.1 to 5 weight parts of (d) an aluminium oxide powder,
0.1 to 7 weight parts of (e) a sensitizer,
10 to 45 weight parts of (f) a polymerizable compound not having an acidic group,
10 to 45 weight parts of (g) an organic solvent, and
0.1 to 15 weight parts of (h) a filler other than the aluminium oxide powder,
wherein 0.4 mol equivalent or less of the acidic group of (a) the polymerizable compound having the acidic group and 0.05 mol equivalent or less of a reduction part of (b) the reducing agent are contained per 100 m² of a specific surface area of (d) the aluminium oxide powder.

As to (a) the polymerizable compound having the acidic group being used to the dental composition according to the present invention, it is not especially limited if said compound has the acidic group in its molecule. However, the polymerizable monomer having such an acidic group as a phosphoric acid group, a carboxyl group or the like, is preferable in order to improve the adhesive strength and durability to the tooth. The polymerizable manomer having the phosphoric acid group is the polymerizable monomer having one or plural phosphoric acid groups in one molecule, and since the phosphoric acid group shows stronger acidity than the carboxyl group, the polymerizable monomer having the phosphoric acid group has the excellent effects to the dissolution of a smear layer of a tooth surface and the tooth demineralization, and especially demonstrates the excellent improvement of the adhesion to enamel. As the polymerizable monomer having phosphoric acid groups, the following monomers can be used. They are 2-(meth) acryloyloxyethyl dihydrogen phosphate (it means to 2-acryloyloxyethyl dihydrogen phosphate or 2 - methacryloyloxyethyl dihydrogen phosphate),
3-(meth)acryloyloxypropyl dihydrogen phosphate,
4-(meth)acryloyloxybutyl dihydrogen phosphate,
5-(meth)acryloyloxypentyl dihydrogen phosphate,
6-(meth)acryloyloxyhexyl dihydrogen phosphate,
7-(meth)acryloyloxyheptyl dihydrogen phosphate,
8-(meth)acryloyloxyoctyl dihydrogen phosphate,
9-(meth)acryloyloxynonyl dihydrogen phosphate,
10-(meth)acryloyloxydecyl dihydrogen phosphate,
11-(meth)acryloyloxyundecyl dihydrogen phosphate,
12-(meth)acryloyloxydodecyl dihydrogen phosphate,
16-(meth)acryloyloxyhexadecyl dihydrogen phosphate
18-(meth)acryloyloxyoctadecyl dihydrogen phosphate, 20-(meth)acryloyloxyeicocyl dihydrogen phosphate,
4 - [2 - (meth)acryloyloxyethyl] cyclohexyloxy dihydrogen phosphate,
bis [2 - (meth)acryloyloxyethyl] hydrogen phosphate,
bis[3-(meth) acryloyloxypropyl] hydrogen phosphate,
bis [4 - (meth) acryloyloxybutyl] hydrogen phosphate,
bis [5 - (meth) acryloyloxypentyl] hydrogen phosphate,
bis [6 - (meth) acryloyloxyhexyl] hydrogen phosphate,
bis[7-(meth) acryloyloxyheptyl] hydrogen phosphate,
bis [8 - (meth) acryloyloxyoctyl] hydrogen phosphate,
bis [9 - (meth) acryloyloxynonyl] hydrogen phosphate,
bis[10-(meth)acryloyloxydecyl] hydrogen phosphate,
2-(meth) acryloyloxyethylphenyl hydrogen phosphate,
2-(meth)acryloyloxyethylanisyl hydrogen phosphate,
2-(meth)acryloyloxyethyltrile hydrogen phosphate,
6-(meth)acryloyloxyhexylphenyl hydrogen phosphate,
bis[1-chloromethyl-2-(meth)acryloyloxyethyl] hydrogen phosphate,
2-(meth)acryloyloxyethylhexyl hydrogen phosphate,
2-(meth)acryloyloxyethyl-2'-bromooctyl hydrogen phosphate,
2-(meth)acryloyloxyethyloctyl hydrogen phosphate,
2-(meth) acryloyloxyethylnonyl hydrogen phosphate,
2-(meth)acryloyloxyethyldecyl hydrogen phosphate,
2-(meth)acryloyloxybutyldecyl hydrogen phosphate,
1,3-di(meth)acryloylpropane-2-dihydrogen phosphate,
1,3-di(meth)acryloylpropane-2-phenylhydrogen phosphate,
bis[5-{2-(meth)acryloyloxyethoxycarbonyl)pentyl] hydrogen phosphate or the like. In these polymerizable monomers having phosphoric acid groups, 10-(meth) acryloyloxydecyl dihydrogen phosphate is especially preferable in the point of view of the adhesiveness and the stability of the monomer itself. These polymerizable monomers having phosphoric acid groups may be used independently or by mixing two or more.

The polymerizable monomer having the carboxyl group is the polymerizable monomer having plural carboxyl groups in one molecule or generating plural carboxyl groups in one molecule by easily reacting with water, and said polymerizable monomer has especially high improvement effect of the adhesion to the dentin since the carboxyl group reacts mildly as compared with the phosphoric acid group and a risk of collagen denaturation of the dentin is less. As the polymerizable monomer having plural carboxyl groups in one molecule or generating plural carboxyl groups in one molecule by easily reacting with water, the following monomers can be used. They are
4-(meth)acryloyloxyethyltrimellitic acid,
4-(meth)acryloyloxyethyltrimellitic anhydride,
4-(meth)acryloyloxydecyltrimellitic acid,
4-(meth) acryloyloxydecyltrimellitic anhydride,
11- (meth) acryloyloxyundecane-1, 1-dicarboxylic acid,
1, 4 di(meth)acryloyloxyethylpyromellitic acid,
2-(meth) acryloyloxyethylmaleic acid,
2-(meth)acryloyloxyethylphthalic acid,
2-(meth)acryloyloxyethylhexahydrophthalic acid,
6-(methacryloyloxyethylnaphthalene-1,2,6-tricarboxylic acid or the like. These polymerizable monomers having the carboxyl group may be used independently or by mixing two or more. In these polymerizable monomers, 4 - (meth) acryloyloxyethyltrimellitic acid and 4 - (meth) acryloyloxyethyltrimellitic anhydride are especially preferable in the point of view of the adhesion.

As the polymerizable monomer having the other acidic group, the following monomers can be used. They are a polymerizable monomer having a phosphonic acid group, such as 2 -(meth) acryloyloxyethylphenylphosphonate or the like, a polymerizable monomer having a thiophosphoric acid group, such as 2-(meth)acryloxyethyl dihydrogen thiophosphate,
3-(meth)acryloyloxypropyl dihydrogen thiophosphate,
4-(meth)acryloyloxybutyl dihydrogen thiophosphate,
6-(meth)acryloyloxyhexyl dihydrogen thiophosphate,
8-(meth)acryloyloxyoctyl dihydrogen thiophosphate,
10-(meth)acryloyloxydecyl dihydrogen thiophosphate,
12-(meth)acryloyloxydodecyldihydrogenthiophosphate,
16-(meth)acryloyloxyhexadecyl dihydrogen thiophosphate,
18 - (meth) acryloyloxyoctadecyl dihydrogen thiophosphate,
20 - (meth) acryloyloxyoeicosyl dihydrogen thiophosphate,
1, 3 - di (meth) acryloyloxypropane - 2 - dihydrogen thiophosphate,
2 - (meth) acryloyloxyethylphenyl hydrogen thiophosphate,
2-(meth)acryloyloxyethylanisyl hydrogen thiophosphate,
2-(meth)acryloyloxyethyltolyl hydrogen thiophosphate or the like, a polymerizable monomer having a thiophosphonic acid group, such as 2-(meth) acryloyloxyethylphenyl thiophosphate or the like, and a polymerizable monomer having a pyrophosphoric acid group, such as di[2-(meth)acryloyloxyethyl] pyrophosphate or the like. These polymerizable monomers can be used independently or by mixing two or more, and also can be used by mixing with other compound if the object of the present invention can be attained.

If the amount of (a) the polymerizable compound having the acidic group is less than 5 weight parts, it is in the tendency that the adhesive property to the tooth becomes weak. On the other hand, if said amount is more than 35 weight parts, it is in the tendency that the adhesion becomes to be decreased.

As (b) the reducing agent used to the dental composition according to the present invention, the tertiary amine and a barbituric acid derivative, which are generally used as the reducing agent in the dental field, can be used in order to increase curability in the adhesion interface and adhesive layers. Further, as the other reducing agent, benzoyloxyperoxide, a sulfinate soda derivative and an organometallic compound or the like can be used. As the tertiary amine kinds, for example, any tertiary amines, such as an aromatic tertiary amine, an aliphatic tertiary amine or the like, can be used. As the aromatic tertiary amine, for example, the following amines can be used. They are N, N-dimethylaniline, N, N-dimethyl-p-toluidine, N,N-diethyl - p-toluidine, N,N-dimethyl-3,5-dimethylaniline, N,N-dimethyl-4-ethylaniline, N,N--dimethyl-4-t-butylaniline, N,N-bis(2-hydroxyethyl)-p-toluidine, N,N-bis(2-hydroxyethyl)-3,5-dimethylaniline, N,N-bis(2-hydroxyethyl)-3,4-dimethylaniline, N,N-bis(2-hydroxyethyl)-4-ethylaniline, N,N-bis(2-hydroxyethyl)-4-t-butylaniline, N, N - bis (2 - hydroxyethyl) - 3, 5 - t - dibutylaniline, ethyl 4-N, N-dimethylamino benzoate, isoamyl 4-N, N-dimethylamino benzoate, n-butoxyethyl 4 - N, N - dimethylamino benzoate, (2 - methacryloyloxy) ethyl 4-N, N-dimethylamino benzoate, 4 -N, N-dimethylamino benzophenone or the like. As the aliphatic tertiary amine, the following amines can be used, that is, trimethylamine, triethylamine, N-methyldiethanolamine, N-ethyldiethanolamine, N-n-butyldiethanolamine, N-lauryldiethanolamine, triethanolamine, (2-dimethylamino) ethylmethacrylate, N-methyldiethanolamine dimethacrylate, N-ethyldiethanolamine dimethacrylate, triethanolamine monomethacrylate, triethanolamine dimethacrylate, triethanolamine trimethacrylate or the like.

As the barbituric acid derivative, the following barbituric acids can be used. They are 1, 3, 5-trimethylbarbituric acid, 1, 3, 5-triethylbarbituric acid, 1,3-dimethyl-5-ethylbarbituric acid, 1, 5-dimethylbarbituric acid, 1-methyl-5-ethylbarbituric acid, 1-methyl-5-propylbarbituric acid, 5-ethylbarbituric acid, 5-propylbarbituric acid, 5-butylbarbituric acid, 1-benzyl-5-phenylbarbituric acid, 1-cyclohexyl-5-ethylbarbituric acid, or the like.

The amount of (b) the reducing agent used to the dental composition according to the present invention is 0.1 to 5 weight parts in the dental composition. If said amount is less than 0.1 weight parts, it is difficult to obtain the effect as the reducing agent. If said amount is more than 5 weight parts, it is in the tendency to decrease the deposition on the tooth tissue at the time of using and the preservation stability of the dental composition. (c) the water used to the dental composition according to the present invention is necessary for carrying out the tooth demineralization or the like by (a) the polymerizable compound having the acidic group through the water, and distilled water, pure water, an ion-exchanged water, deionized water or the like is preferable. The water content is 10 to 55 weight parts in the dental composition. If said content is less than 10 weight parts, the tooth adhesive property of the dental composition decreases. If said amount is more than 55 weight parts, it is in the strong tendency to decrease the polymerzation-curability of the dental composition.

As to (d) the aluminum oxide powder used to the dental composition according to the present invention, it is preferable that the particle size is 0.001 to 50 µm , more preferably 0.001 to 0.1 µm. At this time, it is possible to simultaneously obtain the effect as (h) the filler other than the aluminum oxide powder as mentioning below.

The amount of (d) the aluminum oxide powder used to the dental composition according to the present invention is 0.1 to 5 weight parts in the dental composition. If said amount is less than 0.1 weight parts, it is difficult to obtain the effect to the preservation stability. If said amount is more than 5 weight parts, it is in the tendency to difficultly use the dental composition since its viscosity is influenced and, further, when (d) the aluminum oxide powder is applied to the photopolymerizable dental composition, it is in the tendency to difficultly obtain the curability by a light shielding effect of aluminum oxide powder. Further, in order to obtain the effect of the preservation stability to the maximum extent, 0.4 mol equivalent or less of the acidic group of (a) the polymerizable compound having the acidic group and 0.05 mol equivalent or less of (b) the reducing agent are contained per 100 m² of the specific surface area of (d) the aluminum oxide powder.

At this time, in the case that the acidic group is the carboxylic acid group, the mol equivalent of the acidic group to the specific surface area of the aluminum oxide powder can be calculated then by using the number of the acidic group. However, only in the case that the acidic group is the phosphoric acid group, the mol equivalent is calculated by using the number of -OH directly connecting with P atom. Further, the reduction part of the reducing agent means the radical generating part in the reducing agent, and the mol equivalent of the reduction part to the specific surface area can be calculated by using said radical generating part as one unit. For example, in the case that the reducing agent is the tertiary amine, the mol equivalent can be calculated by using -N (CH₃) as one reduction part. For example, in the case of the barbituric acid derivative, since the number of the radical generating part is one per the barbituric acid derivative, the number itself of the barbituric acid derivative becomes to the number of the reduction part.

In the dental composition according to the present invention, (e) the sensitizer is contained for obtaining the self-curability and stronger adhesion with the tooth. The photopolymerization initiator is suitable as the sensitizer, and for example, a α-diketone compound, a ketal compound, an anthraquinone compound, a thioxanthone compound, a benzoin alkyl ether compound or the like can be effectively used. Further, an acylphosphine oxide compound can be also used together. As the a -diketone compound, for example, camphorquinone, benzyl, diacetyl, acenaphthenequinone, 9, 10 - phenanthraquinone or the like, can be used. As the ketal compound, for example, benzyl dimethyl ketal, benzyl diethyl ketal, benzyl di β-phenylethyl) ketal, benzyl di(2-methoxyethyl) ketal, or the like, can be used. As the anthraquinone compound, for example, anthraquinone, β-methyl anthraquinone, β-ethyl anthraquinone or the like, can be used. As the thioxanthone compound, for example, 2 - ethyl thioxanthone, 2-chloro thioxanthone, 2-hydroxy-3-(3, 4-dimethyl-9-oxo-9H-thioxanthene-2-yloxy) -N, N, N-trimethyl-1- propanaluminum chloride or the like, can be used. As the benzoin alkyl ether compound, for example, benzoin methyl ether, benzoin ethyl ether, benzoin propyl ether, or the like, can be used. In these sensitizers, camphorquinone and benzyl are especially preferable. Further, as the acylphosphine oxide compound, for example, 2, 4, 6 - trimethylbenzoyl diphenylphosphine oxide, 2, 6 - dimethylbenzoyl diphenylphosphine oxide, 2, 6 - dimethoxybenzoyl diphenylphosphine oxide or the like, can be used.

The amount of (e) the sensitizer used to the dental composition according to the present invention is 0.1 to 7 weight parts. If said amounts is less than 0.1 weight parts, it is difficult to obtain a sufficiently sensitizing effect. If said amount is more than 7 weight parts, it is in the tendency to influence to the adhesive property of the dental composition.

In the dental composition to the present invention, (f) the polymerizable compound not having the acidic group is contained further. As (f) the polymerizable compound not having the acidic group, the polymerizable compound conventionally used as a dental material can be used, and a vinyl compound is more preferable. As (f) the polymerizable compound not having the acidic group, the following compounds can be used. They are, for example, methyl(meth)acrylate, ethyl(meth)acrylate, isopropyl (meth) acrylate, n-butyl(meth)acrylate, isobutyl(meth)acrylate, tetrahydrofurfuryl(meth) acrylate, glycidyl(meth)acrylate, 2-methoxyethyl(meth)acrylate, 2-ethylhexyl(meth)acrylate, benzyl(meth)acrylate, 2,2--bis[(meth) acryloyloxyphenyl] propane, 2, 2-bis[4-(meth)acryloyloxydiethoxyphenyl] propane, 2,2-bis[4-(meth)acryloyloxy polyethoxyphenyl] propane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth) acrylate, 1, 6-hexanediol di(meth)acrylate, trimethylolpropane tri(meth) acrylate, pentaerythritol tri(meth)acrylate, trimethylolmethane tri(meth) acrylate, pentaerythritol tetra(meth)acrylate, 2-hydroxyethyl(meth) acrylate, 2-hydroxypropyl(meth)acrylate, 2-hydroxy-1,3-di(meth) acryloyloxypropane, 1,2-dihydroxy-3-(meth)acryloyloxypropane, 2,2-bis(4-(2-hydroxy-3-(meth)acryloyloxypropoxy] phenyl] propane or the like. Further, as the polymerizable monomer not having the acidic group, which has urethane bond in the molecule, di-2-(meth)acryloyloxyethyl-2,2,4-trimethylhexamethylene dicarbamate or the like, can be used.

The amount of (f) the polymerizable compound not having the acidic group, which is used to the dental composition according to the present invention, is 10 to 45 weight parts. If said amount is less than 10 weight parts, the sufficient effect can be difficultly obtained, and if said amount is more than 45 weight parts, the adhesive strength to the tooth is decreased, thus it is not preferable.

As (g) the organic solvent, acetone, methanol, ethanol, isopropyl alcohol, methyl ethyl ketone or the like, which have especially high volatility and hydrophilicity, are preferable. These (g) the organic solvents can be used independently or by mixing two or more. When these (g) the organic solvents are contained, various polymerzable monomers can be easily compatible with water, so that the dental composition having high transparency can be obtained. Further, even if the polymerzable monomer cannot be compatible with water completely because of the containing amounts, the dental composition can be supplied in emulsion state. At this time, when the emulsion state is unstable, for example, it is preferable that a ball shape block material, such as alumina, zirconia or the like is loaded in a container containing the dental composition if necessary, and said container is timely shaken before using. The amount of (g) the organic solvent is 10 to 45 weight parts in the dental composition. If said amount is less than 10 weight parts, it is difficult to obtain the above-mentioned effects. If said amount is more than 45 weight parts, it is in the tendency to decrease the adhesive property to the tooth.

In the dental composition according to the present invention, (h) the fillers other than the aluminum oxide powder are contained in order to improve the adhesive property, coatability, flowability, mechanical strength or the like, if necessary.

The form of the filler other than (h) the aluminum oxide powder is not especially limited, and any form, such as a powdery, plate, sheet, fibrous, porous shape or the like, may be used. To the part applied with the dental composition, the hydrophobicity polymerizable composition, such as the dental composite resin, the dental resin-reinforced type cement or the like, is further applied and filled. So, in order to improve the compatibility of the filler with the dental composite resin, the dental resin-reinforced type cement or the like, the surface treatment, such as a silane treatment, may be carried out if these fillers are an inorganic.

As the material of (h) the filler other than the aluminum oxide powder, the following organic or inorganic materials can be used. They are silica, crystal quartz, a hydroxy apatite, titanium oxide, yttrium oxide, zirconia, calcium phosphate, barium sulfate, aluminium hydroxide, sodium fluoride, a mineral such as kaolin, clay, and mica, ceramics, glass, various crosslinking polymers, and the organic or inorganic composite materials containing above-mentioned inorganic material and crosslinking polymer. As the crosslinking polymer, for example, the granular material obtained by copolymerizing the monofunctional (meta)acrylic ester with the polyfunctional (meta)acrylic ester by using the emulsion polymerization method or the suspension polymerization method, can be used. These crosslinking polymers may be the polymers being swelled when they are mixed with an adhesive composition, such as the polymerizable compound having the acidic group, a film-forming agent, an organic solvent or the like. In these crosslinking polymers, polymers having the swelling degree of 100 % or less is suitably used.

The amount of (h) the filler other than the aluminum oxide powder according to the present invention is 0.1 to 15 weight parts. If said amount is less than 0.1 weight parts, it is difficult to obtain the above-mentioned effects. If said amount is more than 15 weight parts, it is in the tendency to difficultly obtain the objective coatability and flowability.

As a matter of course, the very small amount of an ultraviolet absorber, a colorant, a polymerization inhibitor or the like, may be added to the dental composition of the present invention if necessary.

Hereinafter, although the dental composition according to the present invention will be explained with examples, the present invention is not limited to these examples.

As to each component of (a) to (h), Examples 1 to 21 of the dental compositions were obtained with the containing amounts shown in Table 1, and Comparison Examples 1 to 12 of the dental compositions were obtained with the containing amounts shown in Table 2. Further, as the conventional one work step two components type dental composition, a ready-made product not containing the aluminum oxide powder (AQ Bond made by Sun Medical Co. Ltd.) was used in Comparison Example 13. In addition, each name in Tables is as follows.
PM2 : bis(2-methacryloyloxyethyl) dihydrogen phosphate.
PM21 : bis[5-(2-methacryloyloxyethoxycarbonyl)pentyl) hydrogen phosphate.
Phenyl P : 2-methacryloyloxyethylphenyl hydrogen phosphate.
PM2-C6 : bis(6-methacryloyloxyhexyl) hydrogen phosphate.
PM2 - Cl : bis(1-chloromethyl-2-methacryloyloxyethyl) hydrogen phosphate.
MDP : 10 - methacryloyloxydecyl hydrogen phosphate.
4-MET : 4-methacryloyloxyethyltrimellitic acid.
6-MENT : 6-methacryloyloxyethylnaphthalene-1, 2, 6-tricarboxylic acid.
4-AET : 4-acryloyloxyethyltrimellitic acid.
MAC - 10 : 11-methacryloyloxyundecane-1,1-dicarboxylic acid.
DAAE : ethyl 4-N, N-dimethylaminoethylbenzoate.
CEBA : 1-cyclohexyl-5-ethylbarbituric acid.

Aluminum Oxide Powder : Aluminium oxide C (made by Nippon Aerosil Co. Ltd.).
CQ : camphorquinone
HEMA : 2-hydroxyethylmethacrylate.
GDMA : 2-hydroxy-1, 3-dimethacryloyloxypropane.
TEGDMA : Triethylene glycol dimethacrylate.
UDMA : Di - 2 - methacryloyloxyethyl - 2, 2, 4-trimethylhexamethylene dicarbamate.
A50 : Aerosil 50 (made by Nippon Aerosil Co. Ltd.).
A200 : Aerosil 200 (made by Nippon Aerosil Co. Ltd.).
R972 : Aerosil R972 (made by Nippon Aerosil Co. Ltd.).

### <Preservation stability test>

The dental compositions of each example and comparison example were preserved in a thermostatic apparatus in which the temperature is set to 45 degree C, and the existences of curing of said compositions were confirmed every week with following evaluations. These results were collectively shown in Table 1 and Table 2.
A : Curing of liquid could not be recognized for more than four weeks.
B : Curing of liquid could be recognized in two to four weeks.
C : Curing of liquid could be recognized earlier than two weeks.

### <Adhesive property test 1>

1. The surface of the fresh bovine front tooth was polished with a water resistant polishing sheet of #600 under irrigation so as to expose five each of enamel and dentin surfaces.
2. A fluororesin tape, in which the hole having a diameter of 2.5 mm was open, was stuck on the polished dentin surface or enamel surface to regulate the adhered area. The dental compositions other than those of Examples 1, 2, 19, 20, 21 and Comparison Examples 10 to 12 were applied on the regulated adhered area without carrying out the etching and primer treatments, and after 20 seconds, said compositions were dried with air. Next, said dried compositions were irradiated with light for 10 seconds by a dental visible light irradiator (the product name was GC New Light VLII, made by GC Corporation.).
3. A silicone rubber mold having a height of 2.0 mm, in which the hole having an inner diameter of 5.0 mm was open, was put on the adhesion surface, and a photopolymerization type composite resin (the product name was UniFil S, made by GC Corporation.) was filled in said silicone rubber mold. Then said resin was irradiated with light by the above-mentioned dental visible light irradiator for 40 seconds to be cured.
4. After stored these test pieces in water at 37 degree C for one day, an acrylics rod for tensile test was equipped with an upper part of the test piece, and the tensile test was carried out at crosshead speed of 1.0 min/min by the universal test equipment (the product name was Auto Graph, made by Shimazu Co. Ltd.). These results were collectively shown in Table 1 and Table 2.

### <Adhesive property test 2>

1. The surface of the front bovine tooth was polished like Adhesive property test 1.
2. The fluororesin tape, in which a hole having a diameter of 2.5 mm was open, was stuck on the polished dentin surface or enamel surface to regulate the adhered area. The dental compositions of Examples 1 and 2 in Table 1 were applied to the regulated adhered area, and after keeping those for 10 seconds, were dried with air.
3. The dental adhesives (the product name was UniFil Bond Bonding Material, made by GC Corporation.) was applied on the adhered area, and was irradiated with light by the above-mentioned dental visible light irradiator for 10 seconds.
4. The silicone rubber mold having the height of 2.0 mm, in which the hole having an inner diameter of 5.0 mm was open, was put on the adhesion surface, and a photopolymerization type composite resin (the product name was UniFil S, made by GC Corporation.) was filled in said silicone rubber mold, and then was irradiated with light by the above-mentioned dental visible light irradiator for 40 seconds to be cured.
5. After stored these test pieces in water at 37 degree C for one day, the acrylics rod for the tensile test was equipped with an upper part of the test piece, and the tensile test was carried out at crosshead speed of 1.0 min/min by the universal test equipment (the product name was Auto Graph, made by Shimazu Co. Ltd.). These results were collectively shown in Table 1.

### <Adhesive property test 3>

1. The surface of the front bovine tooth was polished like Adhesive property test 1.
2. The fluororesin tape, in which the hole having a diameter of 3 mm was open, was stuck on the polished dentin surface or enamel surface to regulate the adhered area. The dental compositions of Examples 19 to 21 and Comparison Examples 10 to 12 were coated on the regulated adhered area, and after keeping those for 10 seconds, were dried with air.
3. The silicone rubber mold having the height of 2.0 mm, in which the hole having an inner diameter of 4.0 mm was open, was put on the adhesion surface, and a resin-reinforced type glass ionomer cement (the product name was Fuji IILC, made by GC Corporation.) was filled in said silicone rubber mold, and then was irradiated with light by the above-mentioned dental visible light irradiator for 40 seconds to be cured.
4. After stored these test pieces in water at 37 degree C for one day, an acrylics rod for the tensile test was equipped with an upper part of the test piece, and the tensile test was carried out at crosshead speed of 1.0 min/min by the universal test equipment (the product name was Auto Graph, made by Shimazu Co. Ltd.). These results were collectively shown in Table 1 and Table 2.

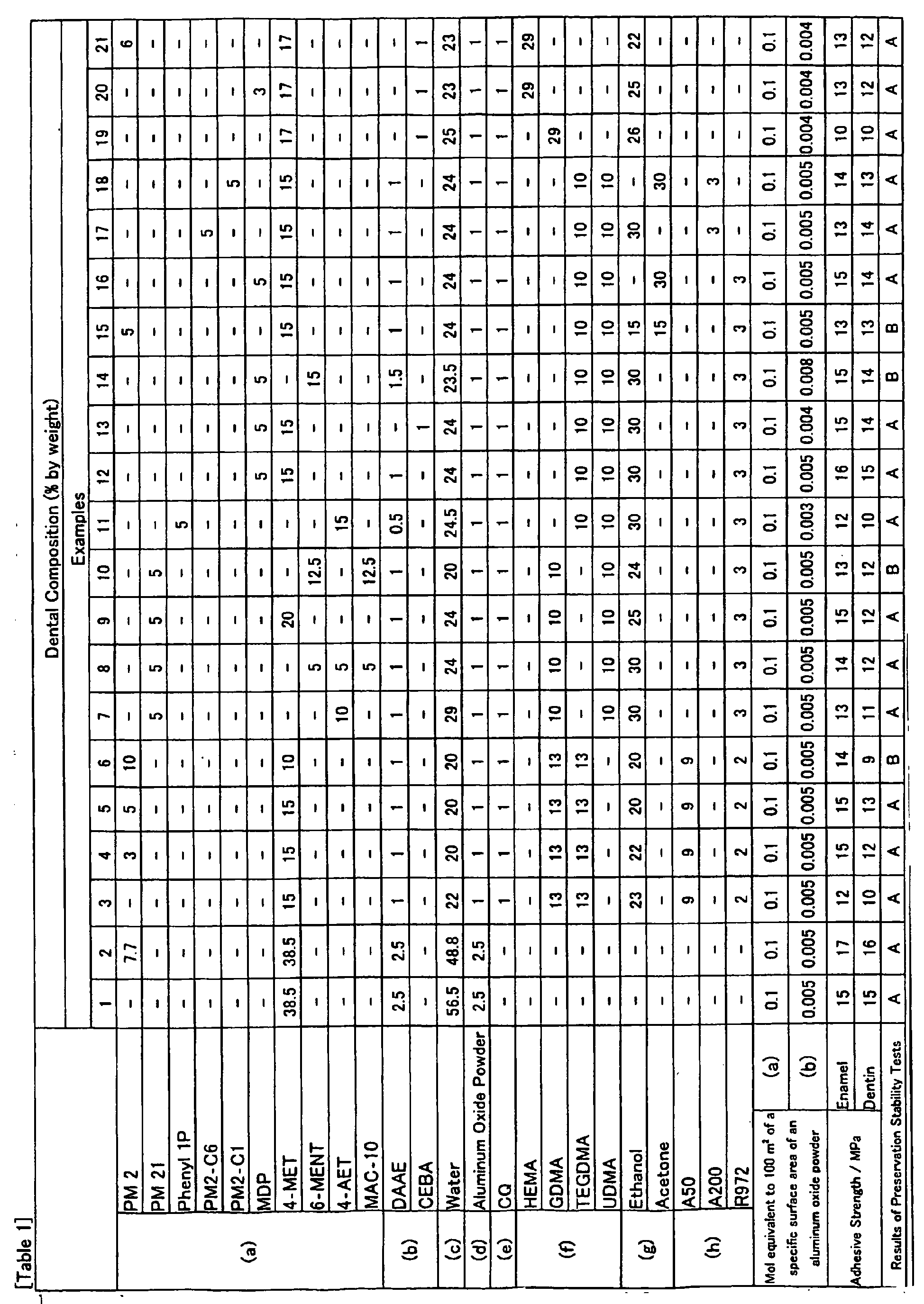

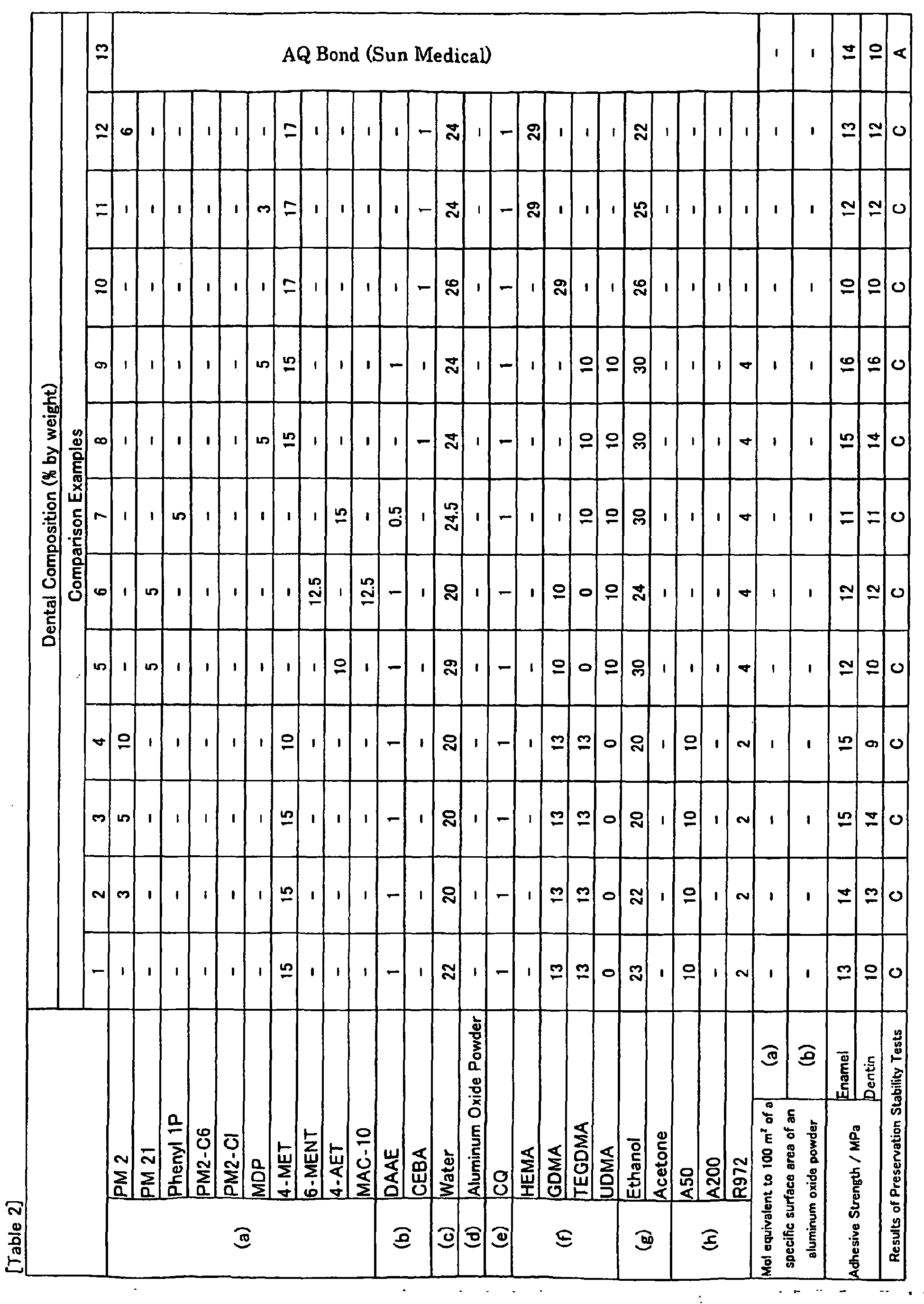

Clearly from the examples and comparison examples, it was confirmed that the dental compositions of the examples were the dental compositions which had the same adhesive property as that of Comparison Example 13 being conventional product but were not cured with time during the period of storage.

As described above, the dental composition according to the present invention is the one work step per one component type dental composition, in which the polymerzable compound having the acidic group is coexisted with the reducing agent such as the tertiary amine or the like under the existence of water, and said dental composition has the same adhesive property as the conventional one but is not cured with time during the period of storage, so that it has the great value for contributing to the field of the dental treatment.

## Claims

1. A dental composition comprising:
5 to 35 weight parts of (a) a polymerizable compound having an acidic group,
0.1 to 5 weight parts of (b) a reducing agent,
10 to 55 weight parts of (c) water,
0.1 to 5 weight parts of (d) an aluminium oxide powder,
0.1 to 7 weight parts of (e) a sensitizer,
10 to 45 weight parts of (f) a polymerizable compound not having an acidic group,
10 to 45 weight parts of (g) an organic solvent, and
0.1 to 15 weight parts of (h) a filler other than the aluminium oxide powder,
wherein 0.4 mol equivalent or less of the acidic group of (a) the polymerizable compound having the acidic group and 0.05 mol equivalent or less of a reduction part of (b) the reducing agent are contained per 100 m² of a specific surface area of (d) the aluminium oxide powder.

## Patentansprüche

1. Dentalzusammensetzung, umfassend:
5 bis 35 Gew.-Teile (a) einer polymerisierbaren Verbindung mit einer Säuregruppe,
0,1 bis 5 Gew.-Teile (b) eines Reduktionsmittels,
10 bis 55 Gew.-Teile (c) Wasser,
0,1 bis 5 Gew.-Teile (d) eines Aluminiumoxidpulvers,
0,1 bis 7 Gew.-Teile (e) eines Sensibilisierungsmittels,
10 bis 45 Gew.-Teile (f) einer polymerisierbaren Verbindung, die keine Säuregruppe aufweist,
10 bis 45 Gew.-Teile (g) eines organischen Lösungsmittels und
0,1 bis 15 Gew.-Teile (h) eines von dem Aluminiumoxidpulver verschiedenen Füllstoffs,
wobei 0,4 Moläquivalente oder weniger der Säuregruppe der (a) polymerisierbaren Verbindung mit einer Säuregruppe und 0,05 Moläquivalente oder weniger eines Reduktionsteils des (b) Reduktionsmittels pro 100 m² eines spezifischen Oberflächenbereichs des (d) Aluminiumoxidpulvers enthalten sind.

## Revendications

1. Composition dentaire comprenant :
de 5 à 35 parties en poids de (a) un composé polymérisable comportant un groupe acide,
de 0,1 à 5 parties en poids de (b) un réducteur,
de 10 à 55 parties en poids de (c) de l'eau,
de 0,1 à 5 parties en poids de (d) une poudre d'oxyde d'aluminium,
de 0,1 à 7 parties en poids de (e) un sensibilisateur,
de 10 à 45 parties en poids de (f) un composé polymérisable ne comportant pas de groupe acide,
de 10 à 45 parties en poids de (g) un solvant organique, et
de 0,1 à 15 parties en poids de (h) une matière de charge autre que la poudre d'oxyde d'aluminium,
dans laquelle un nombre inférieur ou égale à 0,4 mole du groupe acide du composé polymérisable comportant le groupe acide (a) et un nombre inférieur ou égale à 0,05 mole ou moins d'une partie réductrice du réducteur (b) sont contenus pour 100 m² d'une surface spécifique de la poudre d'oxyde d'aluminium (d).
